(19) Europäisches Patentamt
European Patent Office
Office européen des brevets

(11) Publication number: **0 206 322 B1**

# (12) EUROPEAN PATENT SPECIFICATION

(45) Date of publication of patent specification: **26.08.92**

(51) Int. Cl.[5]: **A61K 31/47**, C07D 221/14

(21) Application number: **86108615.5**

(22) Date of filing: **24.06.86**

The file contains technical information submitted after the application was filed and not included in this specification

## (54) N-(2-carboxy)-ethyl-1,8-naphthalene imide and its salts for the treatment of diabetic retinopathies and neuropathies.

(30) Priority: **27.06.85 IT 2132285**

(43) Date of publication of application:
**30.12.86 Bulletin 86/52**

(45) Publication of the grant of the patent:
**26.08.92 Bulletin 92/35**

(84) Designated Contracting States:
**AT BE CH DE FR GB IT LI LU NL SE**

(56) References cited:
**FR-A- 2 521 139**
**US-A- 3 821 383**
**US-A- 4 405 625**

**J. Pharm. Sci., vol.73, no.7, July 1984, pages 956-961; Am. Pharm. Ass. US, I.H.Hall et al.**

**Pharm. Res. no.6, 1984, pages 267-269, J.M.Chapman et al**

(73) Proprietor: **I.P.A. INTERNATIONAL PHARMACEUTICAL ASSOCIATED S.r.l.**
**Via Casale Cavallari, 53**
**I-00156 Rome(IT)**

(72) Inventor: **Malizia, Paolo**
**Via Casale Cavallari, 53**
**I-00156 Rome(IT)**

(74) Representative: **Bianchetti, Giuseppe**
**Studio Consulenza Brevettuale Via Rossini, 8**
**I-20122 Milan(IT)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention refers to pharmaceutical compositions containing, as an active principle, therapeutically effective amounts of non toxic, physiologically acceptable salts of N-(2-carboxy)-ethyl-1,8-naphthaleneimide, of formula I

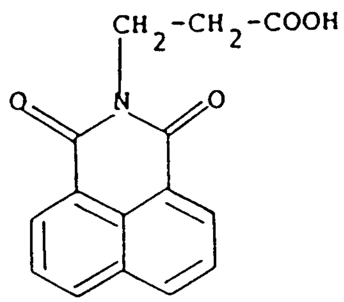

(I)

such as an alkali, earth-alkali, ammonium salt, salts with basic aminoacids such as lysine, ornithine and arginine, with primary, secondary or tertiary amines, or with aminosugars.

The lysine and N-methylglucamine are particularly preferred and provide a further object of the invention.

The compound I is endowed with inhibitory activity of the aldose-reductase, an enzyme responsible for the degradation of hexoses to polyols such as sorbitol and dulcitol.

In the presence of high hexoses levels, as it occurs for instance in the diabetes and in the galactosemias, an accumulation of said polyols occurs in some tissues, and particularly in the eye, at the level of retina and crystalline, lens in the kidneys and in the peripheral nerves, with consequent onset of retinopathies, neuropathies and nephropathies which are the main complications of diabetes.

The compositions containing the compound I are therefore useful in human therapy for the treatment of retinopathies, neuropathies and nephropathies on diabetic basis.

The compound I is known, having been described by El Naggar et al. in Egypt. J. Chem. 24 (2), 127-130 (1981) as endowed with antibacterial activity against Bacillus strains. The compound I is also reported to have hypolipemic activity (J. Pharm. Sci. 1984, 73 (7), 956).

The U.S. Patent No. 3,821,383 discloses pharmaceutical compositions useful in the treatment of diabetes related complications, containing as the active principle the N-(2-carboxy)-methyl-1,8-naphthaleneimide or 1,3-dioxo-1H-benzo[de]isoquinoline-2(3H) acetic acid. Said compound is therefore the lower homolog of the compound I which is however surprisingly more active and endowed with more advantageous pharmaco-therapeutics characteristics.

FR-A-2521139 claims the use of N-(4-carboxy)-propyl-1,8-naphthaleneimide and its salts for the same pathology.

US-A-4405625 claims N-carboxymethyl-1,8-naphthaleneimide for lowering blood glucose in a diabetic mammal.

Pharm.Res. no.6, 1984, p.267-269, shows the antilipidemic activity of various phthahlimide derivatives, including compound I.

US-A-4254109 describes the use of various salts of some derivatives of N-carboxymethyl-1,8-naphthaleneimide for the treatment of diabetic complications.

The compound I hereinbelow also referred to, for the sake of brevity, with the abbreviation **IPA 955**, or one of the salts thereof, may be administered according to the invention by the oral, parenteral or topical route, in form of pharmaceutical compositions containing pharmacologically acceptable excipients and optionally other active principles.

The topical route of administration is particularly important in the ophtalmic field because the etiological role played by aldose-reductase in the cataract with darkening of the crystalline lens and in the retinopathies induced by galactosemia and diabetes, is known. **IPA 955** is therefore particularly useful for the topical administration in the eye in form of collyres comprising sterile solutions buffered to pH 7.2-7.4.

Suitable forms for the oral administration are capsules, tablets, sugar-coated tablets, syrups, solutions. Suitable forms for the parenteral administration are sterile vials or bottles containing the active principle, optionally mixed with a suitable carrier.

The dosage of **IPA 955** will obviously depend on the seriousness of the pathology to be treated and on patient's weight and age; it may generally range, because of the limited toxicity of the compositions of the

invention, from 1 to 100 mg/kg/die by the oral and parenteral route: however, as a rule, lower dosages, from 1 to 50 mg/kg/die, will be generally sufficient. By the topical route it will be generally suited the instillation of 0.1-5% eye-washes (collyrium) 1-2 times a day.

The unitary dosage forms for oral or parenteral administration may comprise from 10 to about 500 mg, administered from 1 to 4 times a day.

The compound I is prepared from β-alanine and 1,8-naphthalic anhydride, according to the following example.

## EXAMPLE 1

10 Grams (1 mole) of 1,8-naphthalic anhydride were added to a 98% solution of β-alanine (5.05 g, 1.1 mole) and sodium hydroxide (2.26 g) in 50 ml of water. After 6 hours at reflux, the mixture was cooled, acidified with diluted HCl and filtered. After washing with $H_2O$ and double recrystallization from ethanol 95°, 6.42 g of **IPA 955** were obtained (m.p. 319-321°C). 5 Grams of said product were dissolved in absolute ethanol, added with the stoichiometric amount of sodium ethoxide in absolute ethanol, were left overnight to react at the room temperature. The precipitated sodium salt is then filtered, washed with absolute methanol and dried at 60°C.
NMR Spectrum ( δ ; $CDCl_3$/DMSO 80/20):
2.43-2.85 (2H, t); 4.10-4.55 (2H, t); 7.45-7.90 (2H, m); 8.00-8.60 (4H, m).

## EXAMPLE 2

1.1 Grams of L-lysine were added to a solution of 2 g of **IPA 955**, prepared according to the Example 1, in 25 ml of 95% ethanol, at room temperature and under stirring. After 4 hours, the mixture was cooled to 0-5°C and the precipitated salt filtered, whose chemico-physical characteristics agree with those expected for the captioned product.

The N-methylglucamine salt was similarly prepared, by using, instead of lysine, 1.5 g of methylglucamine.

The acute toxicity of **IPA 955**, its aldose-reductase inhibiting activity in comparison with quercitrin and its antiaggregant activity in comparison with ticlopidine have been determined.

## Acute toxicity

The acute toxicity of **IPA 955** has been studied in the mouse after oral and intravenous administration. The $LD_{50}$ values were calculated according to the Litchfield and Wilcoxon method (J. Pharm. Exp. Therap. 1949, 96, 99).

**IPA 955**, at the tested doses, proved to be practically devoid of toxicity.

## Aldose-reductase inhibitory activity

Crystallin lenses of adult male rats have been homogenized in 0.1 M phosphate buffer pH 6.2. The surnatant, after centrifugation at 3000 x g for 15 minutes, has been used. The surnatant contained 13 mg/ml of proteins. Quercitrin (Fluka, Buchs) 1 mM dissolved in DMSO: $H_2O$ (1:10) has been used.

The aldose-reductase activity has been tested by the method described by S.D. Varma and J.H. Kinoshita (Biochem. Pharmacol. 25, 2505, 1976) using 1 ml of final volume containing NAPDH (20 µl of a 10 mM solution), DL glyceraldheide 1.5 x $10^{-3}$ M final, $LiCl_2$ (25 µl of a 1 M solution) and 150 µl of a surnatant. The blank contained the same substances, except the surnatant. As a reference drug, quercitrin, a known aldose-reductase inhibitor, has been used.

**IPA 955**, with a Ki of 1.04 µM, proved to have an activity about 6 times higher than that of quercitrin (Ki = 6.00 µM).

## "In vitro" platelet antiaggregant activity

The platelet antiaggregant activity of **IPA 955** has been determined in comparison with a known antiaggregant activity, Ticlopidine, on the platelets of rabbit blood added with 3.6% sodium citrate in an amount of 10%. The blood was centrifuged for 15' at 1200 rpm and the surnatant was recovered for its platelet content. The residue has been further centrifuged with the aim of obtaining a "poor" plasma (3000 - 3500 rpm). The plasma platelets obtained after the first centrifugation were suitably diluted with the poor

plasma, counted and the rich plasma (first centrifugation) was diluted according to the platelet count so as to obtain a final concentration of platelets of $25.10^4 \div 30.10^4/mm^3$.

The aggregation was induced by the addition of ADP and collagene at the specified concentrations.

The results are reported in the following Tables.

ADP-INDUCED AGGREGATION

Percent inhibition

| Compounds | Dose | conc. 5.0 µM | conc. 6.25 µM | conc. 8.3 µM | conc. 12.5 µM |
|---|---|---|---|---|---|
| IPA 955 | 0.625 mM | | 8.4% | | 23.17% |
| TICLOPIDINE | | | 9.7% | | 15.66% |
| IPA 955 | 1.25 mM | 14.6% | 25.66% | 16.3% | 16.85% |
| TICLOPIDINE | | 24.4% | 20.5% | 19.75% | 20.02% |

| AGGREGATION BY COLLAGENE | | | |
|---|---|---|---|
| | | Percent inhibition | |
| Compounds | Dose | 25 µg/ml | 50 µg/ml |
| IPA 955 | 0.625 mM | 41.05% | |
| TICLOPIDINE | | 45.26% | |
| IPA 955 | 1.25 mM | 48.83% | 38.66% |
| TICLOPIDINE | | 66.5% | 59.6% |

According to the values reported in the Tables, **IPA 955** turns out to be endowed with an high platelet antiaggregation activity, ranging from 40 to 50% in the case of collagen induced aggregation; in the ADP-induced aggregation the percent inhibition is about 20%; in both cases practically comparable, even if slightly lower than that of Ticlopidine.

**Claims**

**Claims for the following Contracting States : BE, CH, DE, FR, GB, IT, LI, LU, NL, SE**

1. Pharmaceutical compositions for the therapy of diabetic neuropathies, retinopathies and nephropathies containing as the active principle non toxic, physiologically acceptable salts of N-(2-carboxy)-ethyl-1,8-naphthaleneimide of formula I

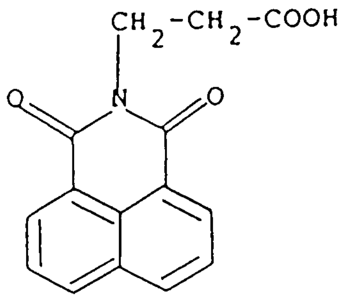

(I)

2. Compositions according to claim 1, containing as the active principle the lysine or N-methylglucamine salt of compound I.

3. Compositions according to claim 1 or 2 for the oral, parenteral or topical administration in form of capsules, tablets, sugar-coated tablets, syrups, solutions, sterile eye washes, collyriums, vials.

4. Compositions according to claims 1, 2 or 3 containing from 10 to 500 mg of the compound I or the equivalent of the salt thereof for the oral or parenteral forms and from 0.1 to 5% of active principle for the topical forms.

5. N-(2-Carboxy)-ethyl-1,8-naphthaleneimide lysine salt.

6. N-(2-Carboxy)-ethyl-1,8-naphthaleneimide N-methylglucamine salt.

7. Use of N-(2-carboxy)-ethyl-1,8-naphthaleneimide or of a non toxic salt thereof for the preparation of a medicament useful for the therapeutic or prophylactic treatment of diabetic neuropathies, retinopathies and nephropathies.

**Claims for the following Contracting State : AT**

1. Process for the preparation of pharmaceutical compositions for the therapy of diabetic neuropathies, retinopathies and nephropathies characterized by admixing N-(2-carboxy)-ethyl-1,8-naphthaleneimide of formula I

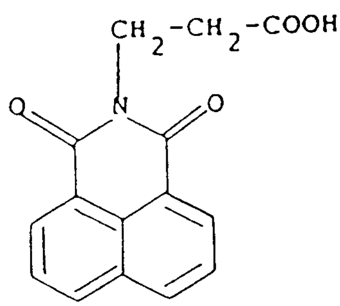

(I)

with suitable, non toxic excipients.

2. Process for the preparation of N-(2-carboxy)-ethyl-1,8-naphthaleneimide lysine or N-methylglucamine salt characterized in that N-(2-carboxy)-ethyl-1,8-naphthaleneimide is reacted with substantially equimolar amounts of L-lysine or N-methylglucamine in the presence of a solvent.

**Patentansprüche**

**Patentansprüche für folgende Vertragsstaaten : BE, CH, DE, FR, GB, IT, LI, LU, NL, SE**

1. Arzneimittel zur Therapie von diabetischen Nervenleiden, Netzhauterkrankungen und Nierenleiden, die als Wirkstoff ungiftige, physiologisch verträgliche Salze des N-(2-Carboxy)-ethyl-1,8-naphtalinimids der

Formel (I)

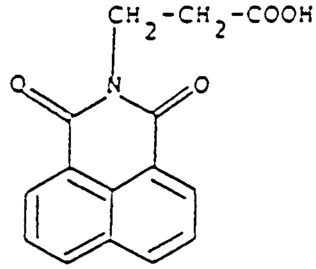

(I)

enthalten.

2. Mittel gemäß Anspruch 1, die als Wirkstoff das Lysin- oder N-Methylglucamin-Salz der Verbindung (I) enthalten.

3. Mittel gemäß Anspruch 1 oder 2, zur oralen, parenteralen oder topischen Verabreichung in Form von Kapseln, Tabletten, Tabletten mit Zuckerüberzug, Sirupen, Auflösungen, sterilen Augenwaschungen, Augenwässern oder Ampullen.

4. Mittel gemäß den Ansprüchen 1, 2 oder 3, enthaltend 10 bis 500 mg der Verbindung (I) oder eine äquivalente Menge von deren Salz für die orale oder parenterale Verabreichungsform, und 0,1 bis 5 % Wirkstoff für die topische Verabreichungsform.

5. Lysinsalz des N-(2-Carboxy)-ethyl-1,8-naphthalinimids.

6. N-Methylglucaminsalz des N-(2-Carboxy)-ethyl-1,8-naphthalinimids.

7. Verwendung des N-(2-Carboxy)-ethyl-1,8-naphthalinimids oder eines ungiftigen Salzes davon zur Herstellung eines Medikaments zur therapeutischen oder prophylaktischen Behandlung von diabetischen Nervenleiden, Netzhauterkrankungen und Nierenleiden.

**Patentansprüche für folgenden Vertragsstaat : AT**

1. Verfahren zur Herstellung von Arzneimitteln zur Behandlung von diabetischen Nervenleiden, Netzhauterkrankungen und Nierenleiden, gekennzeichnet durch das Vermischen von N-(2-Carboxy)-ethyl-1,8-naphtalinimid der Formel (I)

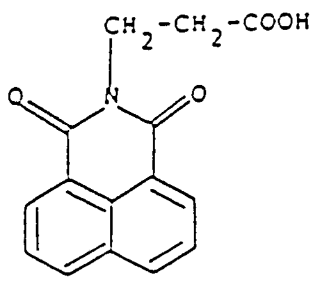

(I)

mit geeigneten ungiftigen Trägerstoffen.

2. Verfahren zur Herstellung des Lysin- oder N-Methylglucaminsalzes des N-(2-Carboxy)-ethyl-1,8-naphthalinimids, dadurch gekennzeichnet, daß das N-(2-Carboxy)-ethyl-1,8-naphthalinimid in Gegenwart eines Lösungsmittels mit im wesentlichen äquimolaren Mengen von L-Lysin oder N-Methylglucamin umgesetzt wird.

**Revendications**

**Revendications pour les Etats contractants suivants : BE, CH, DE, FR, GB, IT, LI, LU, NL, SE.**

1. Compositions pharmaceutiques pour le traitement des nevropathies, rétinopathies et néphropathies diabétiques, contenant, comme principe actif, des sels non toxiques et physiologiquement acceptables de N-(2-carboxy)-éthyl-1,8-naphtalène-imide de formule I

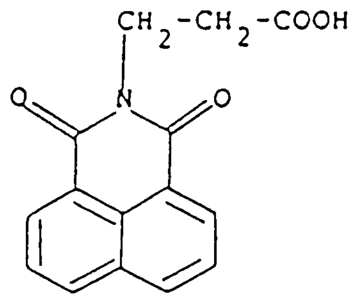

(I)

2. Compositions selon la revendication 1, contenant, comme principe actif, le sel de lysine ou de N-méthylglucamine du composé I.

3. Compositions selon la revendication 1 ou 2, pour une administration orale, parentérale ou topique, sous la forme de capsules, de comprimés, de comprimés dragéifiés, de sirops, de solutions, de lotions ophtalmologiques stériles, de collyres ou en flacons.

4. Compositions selon la revendication 1, 2 ou 3, contenant de 10 à 500 mg du composé I, ou l'équivalent du sel de celui-ci, pour les formes orales ou parentérales, et de 0,1 à 5 % de principe actif pour les formes topiques.

5. Sel de lysine du N-(2-carboxy)-éthyl-1,8-naphtalène-imide.

6. Sel de N-méthylglucamine du N-(2-carboxy)-éthyl-1,8-naphtalène-imide.

7. Utilisation du N-(2-carboxy)-éthyl-1,8-naphtalène-imide ou d'un sel non toxique de celui-ci, pour la préparation d'un médicament pouvant être utilisé pour le traitement thérapeutique ou prophylactique des nevropathies, rétinopathies et néphropathies diabétiques.

**Revendications pour l'Etat contractant : AT**

1. Procédé de préparation de compositions pharmaceutiques pour le traitement des nevropathies, rétinopathies et néphropathies diabétiques, caractérisé en ce qu'on mélange le N-(2-carboxy)-éthyl-1,8-naphtalène-imide de formule I

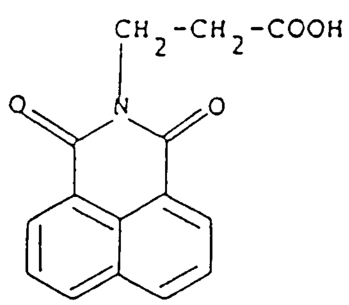

(I)

avec des excipients non toxiques appropriés.

2. Procédé de préparation de sel de lysine ou de N-méthylglucamine du N-(2-carboxy)-éthyl-1,8-naphtalène-imide, caractérisé en ce qu'on fait réagir le N-(2-carboxy)-éthyl-1,8-naphtalène-imide avec des quantités substantiellement équimolaires de L-lysine ou de N-méthylglucamine, en présence d'un solvant.